(19) Europäisches Patentamt
European Patent Office
Office européen des brevets

(11) Numéro de publication: **0 168 288**
**B1**

(12) **FASCICULE DE BREVET EUROPEEN**

(45) Date de publication du fascicule du brevet: **25.04.90**

(21) Numéro de dépôt: **85401119.4**

(22) Date de dépôt: **07.06.85**

(51) Int. Cl.$^5$: **C 07 D 491/048,**
A 61 K 31/435 //
(C07D491/048, 307:00,
221:00)

# (54) Nouveaux dérivés de l'aminométhyl-6 furo-(3,4-c)-pyridine, leur procédé de préparation et compositions thérapeutiques les contenant.

(30) Priorité: **07.06.84 GB 8414559**

(43) Date de publication de la demande:
**15.01.86 Bulletin 86/03**

(45) Mention de la délivrance du brevet:
**25.04.90 Bulletin 90/17**

(84) Etats contractants désignés:
**AT BE CH DE GB IT LI LU NL SE**

(56) Documents cités:
**GB-A-2 092 586**
**GB-A-2 164 037**

**CHEMICAL ABSTRACTS, vol. 101, no. 15, 8 octobre 1984, page 35, no. 122749a, Columbus, Ohio, US; R.P. GARAY et al.: "Stimulation of potassium fluxes by diuretic drugs in human red cells" & BIOCHEM. PHARMACOL. 1984, 33(13), 2013-20**

(73) Titulaire: **SOCIETE DE CONSEILS DE RECHERCHES ET D'APPLICATIONS SCIENTIFIQUES (S.C.R.A.S.)**
**51/53 rue du Docteur Blanche**
**F-75016 Paris (FR)**

(72) Inventeur: **Esanu, André**
**5 avenue d'Erlanger**
**F-75016 Paris (FR)**

(74) Mandataire: **Lachassagne, Jacques**
**Boite Postale 07**
**F-78430 Louveciennes (FR)**

**EP 0 168 288 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne des dérivés de la aminométhyle-6 furo-(3,4-c)-pyridine, un procédé pour la préparation de ces corps ainsi que des compositions thérapeutiques en contenant.

L'invention concerne, plus particulièrement, les dérivés de la dihydro-1,3 aminométhyle-6 hydroxy-7 furo-(3,4-c)-pyridine répondant à la formule générale I

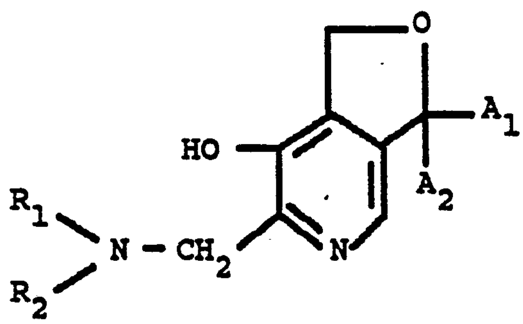

I

dans laquelle chacun des $A_1$ et $A_2$, indépendamment, représente un atome d'hydrogène, un groupement hydrocarboné saturé ou non saturé en chaîne droite ayant de 1 à 5 atomes de carbone, un groupement hétérocyclique ayant jusqu'à 6 atomes dans le cycle, chacun des groupements représentés par $A_1$ et $A_2$ étant non substitué ou substitué par un ou plusieurs atomes de chlore ou de fluor, groupements trifluorométhyle, groupements alcoyle ayant de 1 à 5 atomes de carbone, groupements alcoxy ayant de 1 à 5 atomes de carbone, groupements alcoylthio ayant de 1 à 5 atomes de carbone, groupements dialcoylamino dans lesquels chacun des groupements alcoyle a de 1 à 5 atomes de carbone, groupements dialcoylaminoalcoxy dans lesquels chacun des groupements alcoyle et des groupements alcoxy a de 1 à 5 atomes de carbone ou des groupements α- ou β-alcoxy-N-pyrrolidinyle dans lesquels le groupement alcoxy a de 1 à 5 atomes de carbone; $R_1$ ou $R_2$ représentent l'un un atome d'hydrogène, l'autre un groupement méthyle ou un groupement éthyle, ou $R_1$ et $R_2$, indépendamment, représentent un groupement méthyle ou un groupement éthyle, ou, ensemble avec l'atome d'azote forment un cycle pyrrolidine ou pipéridine ou morpholine ou thiomorpholine; l'invention concerne également les dihydro-1,3 phényle-3 diméthylaminométhyle-6 hydroxy-7 furo-(3,4-c)-pyridine, dihydro-1,3 p-chlorophényle-3 diméthylaminométhyle-6 hydroxy-7 furo-(3,4-c)-pyridine, dihydro-1,3 p-fluorophényle-3 diméthylaminométhyle-6 hydroxy-7 furo-(3,4-c)-pyridine, dihydro-1,3 phényle-3 méthylaminométhyle-6 hydroxy-7 furo-(3,4-c)-pyridine, dihydro-1,3 p-chlorophényle-3 méthylaminométhyle-6 hydroxy-7 furo-(3,4-c)-pyridine, dihydro-1,3 p-fluorophényle-3 méthylaminométhyle-6 hydroxy-7 furo-(3,4-c)-pyridine, dihydro-1,3 phényle-3 pyrrolidinométhyle-6 hydroxy-7 furo-(3,4-c)-pyridine, dihydro-1,3 phényle-3 pipéridinométhyle-6 hydroxy-7 furo-(3,4-c)-pyridine, dihydro-1,3 phényle-3 morpholinométhyle-6 hydroxy-7 furo-(3,4-c)-pyridine, dihydro-1,3 phényle-3 thiomorpholinométhyle-6 hydroxy-7 furo-(3,4-c)-pyridine, dihydro-1,3 éthyle-3 p-chlorophényle-3 diméthylaminométhyle-6 hydroxy-7 furo-(3,4-c)-pyridine, dihydro-1,3 phényle-3 p-fluorophényle-3 diméthylaminométhyle-6 hydroxy-7 furo-(3,4-c)-pyridine, dihydro-1,3 α-furyle-3 phényle-3 méthylaminométhyle-6 hydroxy-7 furo-(3,4-c)-pyridine, dihydro-1,3 p-trifluorométhylphényle-3 p-chlorophényle-3 méthylaminométhyle-6 hydroxy-7 furo-(3,4-c)-pyridine, dihydro-1,3 α-thiényle-3 p-fluorophényle-3 méthylaminométhyle-6 hydroxy-7 furo-(3,4-c)-pyridine, dihydro-1,3 cyclohexyle-3 phényle-3 pyrrolidinométhyle-6 hydroxy-7 furo-(3,4-c)-pyridine, dihydro-1,3 méthyle-3 p-diméthylaminoéthoxyphényle-3 pipéridinométhyle-6 hydroxy-7 furo-(3,4-c)-pyridine, dihydro-1,3 n-pentyle-3 (triméthoxy-3,4,5)-phényle-3 thiomorpholinométhyle-6 hydroxy-7 furo-(3,4-c)-pyridine, dihydro-1,3 phényle-3 (triméthoxy-3,4,5)-phényléthyle-3 diméthylaminométhyle-6 hydroxy-7 furo-(3,4-c)-pyridine, dihydro-1,3 méthyle-3 (p-pyrrolidinyléthoxy)-phényle-3 diméthylaminométhyle-6 hydroxy-7 furo-(3,4-c)-pyridine, dihydro-1,3 p-toluyle-3 p-thiométhylphényle-3 pyrrolidinométhyle-6 hydroxy-7 furo-(3,4-c)-pyridine et leurs sels pharmaceutiquement acceptables.

Les composés selon l'invention présentent un intérêt pour leur activité thérapeutique, notamment dans le domaine de l'action anti-allergique.

Des composés présentant une certaine parenté chimique ont déjà été décrits, notamment dans notre brevet No. 2 092 586, mais du fait de la différence de nature du substituant en position 6, ils ne présentent aucune action dans le domaine anti-allergique.

L'invention concerne, donc, des compositions thérapeutiques qui contiennent au moins un dérivé de la dihydro-1,3 aminométhyle-6 hydroxy-7 furo-(3,4-c)-pyridine répondant à la formule générale I indiquée plus haut ou un sel pharmaceutiquement acceptable de ces dérivés associé à tout diluant ou excipient pharmaceutiquement acceptable.

L'invention concerne, également, un procédé de préparation des composés ci-dessus, ce procédé consistant à faire réagir un dérivé chlorométhyle-6 benzoxy-7 répondant à la formule générale II

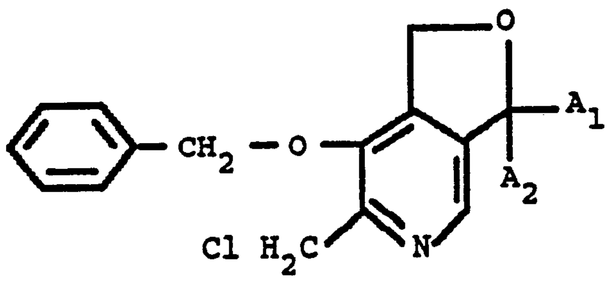

II

dans laquelle $A_1$ et $A_2$ ont la même signification que ci-dessus, avec un excès d'un dérivé aminé répondant à la formule générale IV:

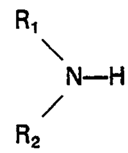

IV

dans laquelle $R_1$ et $R_2$ ont la même signification que ci-dessus, dans un solvant non polaire, à une température ne dépassant pas 20°C, ce qui conduit au dérivé portant la substitution désirée en position 6, puis à traiter ce composé par un acide pour débenzyliser le composé. L'excès du dérivé aminé IV est nécessaire car cette famille de composés comprend des gaz et des liquides à bas point d'ébullition.

Les dérivés chlorométhyle-6 benzoxy-7 II peuvent être obtenus à partir des dérivés méthyle-6 hydroxy-7 répondant à la formule générale III

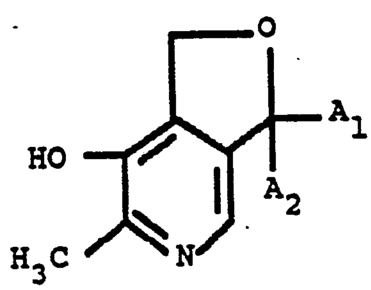

III

dans laquelle $A_1$ et $A_2$ ont la même signification que ci-dessus par la séquence de réactions suivante:

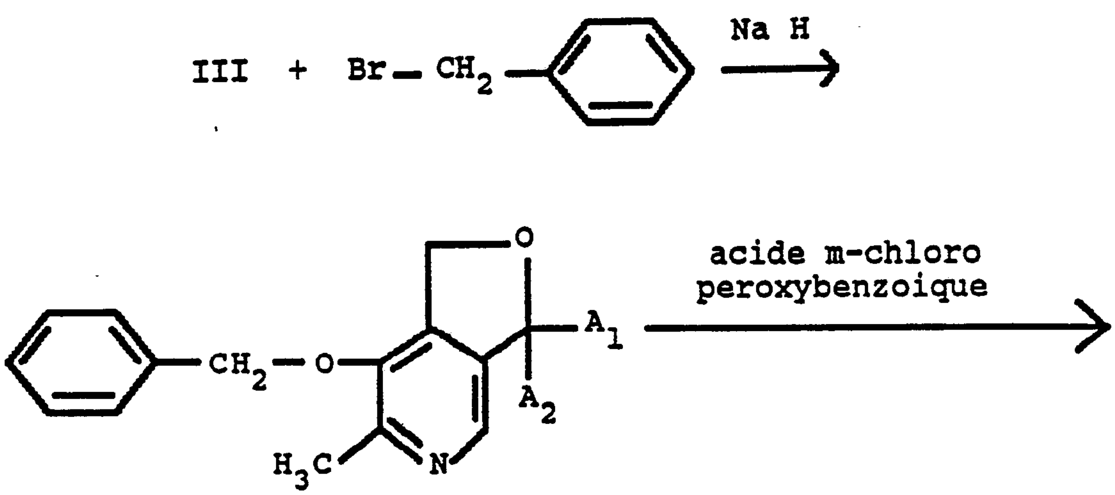

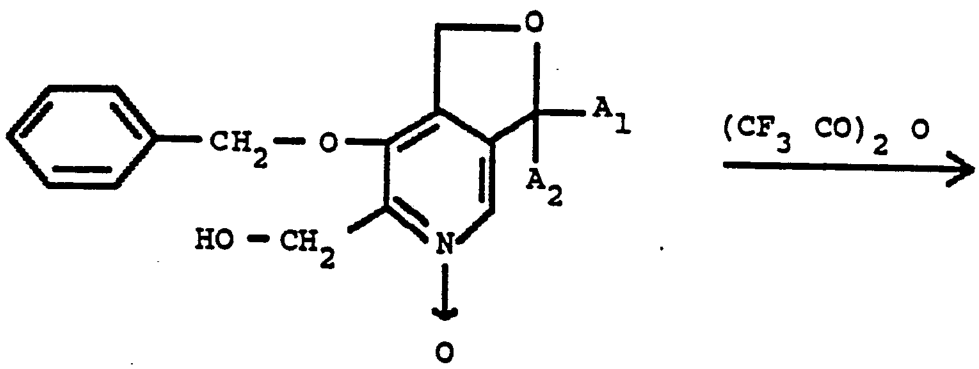


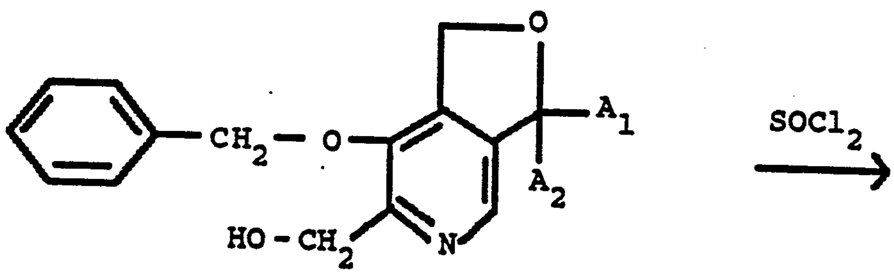


II

Les composés II sont décrits dans nos brevets français No. 82 02135 du 10.02.82 (publication No. 2 494 574) et No. 84 04806 du 28.03.84 (publication No. 2 543 956).

On décrira en détail la préparation d'un seul des composés de départ, la dihydro-1,3 *p*-chlorophényle-3 chlorométhyle-6 benzoxy-7 furo-(3,4-c)-pyridine, les autres produits de départ étant obtenus de la même façon.

a) Dans un réacteur d'un litre équipé avec des moyens d'agitation, de chauffage et de refroidissement, on verse 400 ml de diméthylformamide, 12,5 g d'hydrure de sodium à 50% puis, lentement, sous agitation, 38 g de dihydro-1,3 *p*-chlorophényle-3 méthyle-6 hydroxy-7 furo-(3,4-c)-pyridine. Après une agitation de 90 minutes à température ambiante, on ajoute alors 16 ml de bromure de benzyl et la suspension résultante est maintenue sous agitation pendant une nuit. Après évaporation à sec, le produit pâteux obtenu est agité avec un litre de dichlorure de méthylène, lavé à l'eau jusqu'à élimination complète du chlore et du bromure puis séché sur du sulfate de sodium anhydre. Le dichlorure de méthylène est ensuite évaporé à sec puis le résidu dissous dans l'isopropanol à ébullition, traité au noir de carbone, filtré à chaud et enfin recristallisé. Le produit obtenu est séparé, lavé à l'éther de pétrole puis séché. Rendement 33 g (74%) de dihydro-1,3 *p*-chlorophényle-3 méthyle-6 benzoxy-7 furo-(3,4-c)-pyridine.

b) Dans le même réacteur que ci-dessus, on traite 30 g du produit obtenu à l'étape précédente à 0°C, en présence de 300 ml de dichlorure de méthylène par 18,2 g d'acide m-peroxybenzoique, lentement ajouté. Après avoir laissé le mélange sous agitation pendant une nuit à température ambiante, on ajoute 150 ml d'une solution à 10% de sulfite de sodium. Après agitation et décantation, on lave le produit par la même quantité de sulfite de sodium puis deux fois par 150 ml d'une solution de bicarbonate de soude puis trois fois par 100 ml d'eau et, enfin, on sèche ce produit sur sulfate de sodium anhydre. Par évaporation à sec, on obtient un précipité beige, qui est lavé à l'éther de pétrole et filtré puis séché. Rendement 28 g (90%) de dihydro-1,3 *p*-chlorophényle-3 méthyle-6 benzoxy-7 furo-(3,4-c)-pyridine N-oxyde.

c) Dans le même réacteur que ci-dessus, on traite 28 g du composé obtenu à l'étape précédente, à une température comprise entre 0°C et 5°C, en présence de 175 ml de dichlorure de méthylène par 4,3 ml d'anhydride trifluoroacétique, ajouté goutte à goutte sous agitation. Le mélange réactionnel est soumis à agitation pendant une nuit à température ambiante puis refroidi et additionné de 95 ml de méthanol ajouté goutte à goutte. Après évaporation à sec, le résidu est repris par 300 ml de chloroforme, lavé deux fois par 75 ml d'une solution à 10% de bicarbonate de soude; trois fois par 100 ml d'eau puis séché sur sulfate de sodium anhydre. On évapore, alors, à sec le chloroforme et le résidu est lavé à l'éther éthylique puis séché sous pression réduite. Rendement 25 g (89%) de dihydro-1,3 *p*-chlorophényle-3 hydroxyméthyle-6 benzoxy-7 furo-(3,4-c)-pyridine.

d) Dans un réacteur de deux litres équipé comme l'appareil précédent et placé sous circulation d'azote, on verse 25 g du produit obtenu à l'étape précédente, agité dans 400 ml de benzène sec; on ajoute alors, lentement, 6,3 ml de chlorure de thionyle, sous agitation et à température ambiante. Le mélange réactionnel est alors chauffé à 70°C pendant une heure, ce qui donne un précipité jaune. Ce précipité est alors séparé, lavé au benzène et puis à l'éther éthylique, puis dissous dans 400 ml de chlorure de méthylène. La solution est ensuite lavée par une solution à 10% de bicarbonate de soude jusqu'à pH 8, puis

lavée à l'eau, traitée au noir de carbone, filtrée et concentrée jusqu'à recristallisation. On sépare alors le produit qui est lavé à l'éther éthylique, séché, ce qui donne 254 g (92%) de dihydro-1,3 *p*-chlorophényle-3 chlorométhyle-6 benzoxy-7 furo-(3,4-c)-pyridine.

L'invention sera, d'ailleurs, mieux comprise grâce à la description des exemples qui suivent:

Exemple 1

Dihydro-1,3 méthyle-3 diméthylaminométhyle-6 hydroxy-7 furo-(3,4-c)-pyridine

En utilisant le même appareillage qu'indiqué plus haut, on met en suspension 14,5 g (0.05 mole) de dihydro-1,3 méthyle-3 chlorométhyle-6 benzoxy-7 furo-(3,4-c)-pyridine dans 100 ml de benzène, à 10°C. On ajoute ensuite une solution de 10 ml de diméthylamine dans 100 ml de benzène. Le mélange est soumis à agitation pendant deux heures, ce qui conduit à une solution qui précipite en revenant à la température ambiante. Ce précipité séché est ensuite recristallisé à l'aide de 160 ml d'isopropanol, puis séché. Rendement 87% du dérivé benzoxy correspondant, qui est alors traité par 340 ml d'acide chlorhydrique 2N, sous agitation pendant trois heures. Après évaporation à sec, on obtient 9,29 g de dihydro-1,3 méthyle-3 diméthylamino-6 hydroxy-7 furo-(3,4-c)-pyridine, qui est une substance huileuse. L'analyse élémentaire a montré une bonne correspondance acec la formule $C_{11}H_{16}N_2O_2$, HCl. Le rendement global de cette séquence de réactions est de 76%.

Exemple 2

Dihydro-1,3 propyle-3 méthylaminométhyle-6 hydroxy-7 furo-(3,4-c)-pyridine

On utilise la méthode de l'exemple 1 mais en partant de la dihydro-1,3 propyle-3 chlorométhyle-6 benzoxy-7 furo-(3,4-c)-pyridine et de méthylamine gazeuse dissoute dans le benzène. Le rendement est de 66% d'une substance huileuse dont l'analyse élémentaire a montré une bonne correspondance avec la formule $C_{12}H_{18}N_2O_2$, HCl.

Exemple 3

Dihydro-1,3 phényle-3 diméthylaminométhyle-6 hydroxy-7 furo-(3,4-c)-pyridine

On utilise la méthode de l'exemple 1 mais en partant de la dihydro-1,3 phényle-3 chlorométhyle-6 benzoxy-7 furo-(3,4-c)-pyridine. Le rendement est de 66% d'une substance jaune pâle cristallisée fondant à 196—209°C (Tottoli), dont l'analyse élémentaire a montré une bonne correspondance avec la formule $C_{16}H_{18}N_2O_2$, 2HCl.

Exemple 4

Dihydro-1,3 p-chlorophényle-3 diméthylaminométhyle-6 hydroxy-7 furo-(3,4-c)-pyridine

On utilise la méthode de l'exemple 1 mais en partant de la dihydro-1,3 *p*-chlorophényle-3 chlorométhyle-6 benzoxy-7 furo-(3,4-c)-pyridine. Le rendement est de 49% d'une substance fondant à 207—211°C (Tottoli), dont l'analyse élémentaire a montré une bonne correspondance avec la formule $C_{16}H_{17}N_2O_2Cl$, 2HCl.

Exemple 5

Dihydro-1,3 p-fluorophényle-3 diméthylaminométhyle-6 hydroxy-7 furo-(3,4-c)-pyridine

On utilise la méthode de l'exemple 1 mais en partant de la dihydro-1,3 *p*-fluorophényle-3 chlorométhyle-6 benzoxy-7 furo-(3,4-c)-pyridine. Le rendement est de 57% d'une substance beige clair fondant à 215—220°C (Tottoli), dont l'analyse élémentaire a montré une bonne correspondance avec la formule $C_{16}H_{17}N_2O_2F$, 2HCl.

Exemple 6

Dihydro-1,3 phényle-3 méthylaminométhyle-6 hydroxy-7 furo-(3,4-c)-pyridine

On utilise la méthode de l'exemple 2 mais en partant de la dihydro-1,3 phényle-3 chlorométhyle-6 benzoxy-7 furo-(3,4-c)-pyridine. Le rendement est de 45% d'une substance gris-vert fondant à 190—205°C (Tottoli), dont l'analyse élémentaire a montré une bonne correspondance avec la formule $C_{15}H_{16}N_2O_2$, 2HCl.

Exemple 7

Dihydro-1,3 p-chlorophényle-3 méthylaminométhyle-6 hydroxy-7 furo-(3,4-c)-pyridine

On utilise la méthode de l'exemple 2 mais en partant de la dihydro-1,3 *p*-chlorophényle-3 chlorométhyle-6 benzoxy-7 furo-(3,4-c)-pyridine. Le rendement est de 61% d'une poudre jaune claire fondant à 198°C (Tottoli), dont l'analyse élémentaire a montré une bonne correspondance avec la formule $C_{15}H_{15}N_2O_2Cl$, 2HCl.

Exemple 8

Dihydro-1,3 p-fluorophényle-3 méthylaminométhyle-6 hydroxy-7 furo-(3,4-c)-pyridine

On utilise la méthode de l'exemple 2 mais en partant de la dihydro-1,3 *p*-fluorophényle-3 chlorométhyle-6 benzoxy-7 furo-(3,4-c)-pyridine. Le rendement est de 54% d'une poudre jaune fondant à 228—230°C (Tottoli), dont l'analyse élémentaire a montré une bonne correspondance avec la formule $C_{15}H_{15}N_2O_2F$, 2HCl.

Exemple 9

Dihydro-1,3 phényle-3 pyrrolidinométhyle-6 hydroxy-7 furo-(3,4-c)-pyridine

On utilise la méthode de l'exemple 3 mais en partant de la pyrrolidine au lieu de la diméthylamine. Le rendement est de 77% d'une substance jaune pâle cristallisée fondant à 107°C (Tottoli), dont l'analyse élémentaire a montré une bonne correspondance avec la formule $C_{18}H_{20}N_2O_4$.

Exemple 10

Dihydro-1,3 phényle-3 pipéridinométhyle-6 hydroxy-7 furo-(3,4-c)-pyridine

On utilise la méthode de l'exemple 9 mais en partant de la pipéridine au lieu de la pyrrolidine. Le rendement est de 66% d'une substance beige pâle cristallisée fondant à 138°C (Tottoli), dont l'analyse élémentaire a montré une bonne correspondance avec la formule $C_{19}H_{22}N_2O_2$.

Exemple 11

Dihydro-1,3 phényle-3 morpholinométhyle-6 hydroxy-7 furo-(3,4-c)-pyridine

On utilise la méthode de l'exemple 9 mais en partant de la morpholine au lieu de la pyrrolidine. Le rendement est de 72% d'une poudre blanche fondant à 172°C (Tottoli), dont l'analyse élémentaire a montré une bonne correspondance avec la formule $C_{18}H_{20}N_2O_3$.

Exemple 12

Dihydro-1,3 phényle-3 thiomorpholinométhyle-6 hydroxy-7 furo-(3,4-c)-pyridine

On utilise la méthode de l'exemple 11 mais en partant de la thiomorpholine au lieu de la morpholine. Le rendement est de 58% d'une poudre gris pâle fondant à 185°C (Tottoli), dont l'analyse élémentaire a montré une bonne correspondance avec la formule $C_{18}H_{20}N_2O_2S$.

Exemple 13

Dihydro-1,3 diméthyle-3,3 diméthylaminométhyle-6 hydroxy-7 furo-(3,4-c)-pyridine

On utilise la méthode de l'exemple 1 mais en partant de la dihydro-1,3 diméthyle-3,3 chlorométhyle-6 benzoxy-7 furo-(3,4-c)-pyridine. Le rendement est de 53% d'une substance jaune pâle cristallisée fondant à 175°C (Tottoli), dont l'analyse élémentaire a montré une bonne correspondance avec la formule $C_{13}H_{20}N_2O_2$, 2HCl.

Exemple 14

Dihydro-1,3 méthyle-3 n-butyle-3 diméthylaminométhyle-6 hydroxy-7 furo-(3,4-c)-pyridine

On utilise la méthode de l'exemple 1 mais en partant de la dihydro-1,3 méthyle-3 *n*-butyle-3 chlorométhyle-6 benzoxy-7 furo-(3,4-c)-pyridine et de la diméthylamine dissoute dans le benzène. Le rendement est de 48% d'une substance huileuse, dont l'analyse élémentaire a montré une bonne correspondance avec la formule $C_{14}H_{22}N_2O_2$, HCl.

Exemple 15

Dihydro-1,3 éthyle-3 p-chlorophényle-3 diméthylaminométhyle-6 hydroxy-7 furo-(3,4-c)-pyridine

On utilise la méthode de l'exemple 1 mais en partant de la dihydro-1,3 éthyle-3 *p*-chlorophényle-3 chlorométhyle-6 benzoxy-7 furo-(3,4-c)-pyridine. Le rendement est de 64% d'une substance fondant à 151°C (Tottoli), dont l'analyse élémentaire a montré une bonne correspondance avec la formule $C_{18}H_{21}N_2O_2Cl$, 2HCl.

Exemple 16

Dihydro-1,3 phényle-3 p-fluorophényle-3 diméthylaminométhyle-6 hydroxy-7 furo-(3,4-c)-pyridine

On utilise la méthode de l'exemple 1 mais en partant de la dihydro-1,3 phényle-3 *p*-fluorophényle-3 chlorométhyle-6 benzoxy-7 furo-(3,4-c)-pyridine. Le rendement est de 44% d'une substance beige fondant à 184°C (Tottoli), dont l'analyse élémentaire a montré une bonne correspondance avec la formule $C_{22}H_{21}N_2O_2F$, 2HCl.

Exemple 17

Dihydro-1,3 α-furyle-3 phényle-3 méthylaminométhyle-6 hydroxy-7 furo-(3,4-c)-pyridine

On utilise la méthode de l'exemple 2 mais en partant de la dihydro-1,3 α-furyle-3 phényle-3 chlorométhyle-6 benzoxy-7 furo-(3,4-c)-pyridine. Le rendement est de 42% d'une substance blanche cristallisée fondant à 191—193°C (Tottoli), dont l'analyse élémentaire a montré une bonne correspondance avec la formule $C_{19}H_{18}N_2O_3$, 2HCl.

Exemple 18

Dihydro-1,3 p-trifluorométhylphényle-3 p-chlorophényle-3 méthylaminométhyle-6 hydroxy-7 furo-(3,4-c)-pyridine

On utilise la méthode de l'exemple 3 mais en partant de la dihydro-1,3 *p*-trifluorométhylphényle-3 *p*-chlorophényle-3 chlorométhyle-6 benzoxy-7 furo-(3,4-c)-pyridine. Le rendement est de 59% d'une poudre jaune fondant à 178°C (Tottoli), dont l'analyse élémentaire a montré une bonne correspondance avec la formule $C_{22}H_{19}N_2O_2F_3Cl$, 2HCl.

Exemple 19

Dihydro-1,3 α-thiènyle-3 p-fluorophényle-3 méthylaminométhyle-6 hydroxy-7 furo-(3,4-c)-pyridine

On utilise la méthode de l'exemple 2 mais en partant de la dihydro-1,3 α-thiènyle-3 *p*-fluorophényle-3 chlorométhyle-6 benzoxy-7 furo-(3,4-c)-pyridine. Le rendement est de 55% d'une poudre jaune fondant à 184—189°C (Tottoli), dont l'analyse élémentaire a montré une bonne correspondance avec la formule $C_{19}H_{17}N_2O_2SF$, 2HCl.

Exemple 20

Dihydro-1,3 cyclohexyle-3 phényle-3 pyrrolidinométhyle-6 hydroxy-7 furo-(3,4-c)-pyridine

On utilise la méthode de l'exemple 3 mais en partant de la dihydro-1,3 cyclohexyle-3 phényle-3 chlorométhyle-6 benzoxy-7 furo-(3,4-c)-pyridine et de la pyrrolidine au lieu de diméthylamine. Le rendement est de 67% d'une substance jaune cristallisée fondant à 204°C (Tottoli), dont l'analyse élémentaire a montré une bonne correspondance avec la formule $C_{24}H_{30}N_2O_4$.

Exemple 21

Dihydro-1,3 méthyle-3 p-diméthylaminoéthoxyphényle-3 pipéridinométhyle-6 hydroxy-7 furo-(3,4-c)-pyridine

On utilise la méthode de l'exemple 10 mais en partant de la dihydro-1,3 méthyle-3 *p*-diméthylaminoéthoxyphényle-3 chlorométhyle-6 benzoxy-7 furo-(3,4-c)-pyridine. Le rendement est de 38% d'une substance beige cristallisée fondant à 116°C (Tottoli), dont l'analyse élémentaire a montré une bonne correspondance avec la formule $C_{24}H_{33}N_2O_2$.

Exemple 22

Dihydro-1,3 éthyle-3 diméthylaminopropyle-3 morpholinométhyle-6 hydroxy-7 furo-(3,4-c)-pyridine

On utilise la méthode de l'exemple 11 mais en partant de la dihydro-1,3 éthyle-3 diméthylaminopropyle-3 chlorométhyle-6 benzoxy-7 furo-(3,4-c)-pyridine. Le rendement est de 42% d'une substance blanche fondant à 167°C (Tottoli), dont l'analyse élémentaire a montré une bonne correspondance avec la formule $C_{19}H_{30}N_3O_3$.

Exemple 23

Dihydro-1,3 n-pentyle-3 (triméthoxy-3,4,5)-phényle-3 thiomorpholinométhyle-6 hydroxy-7 furo-(3,4-c)-pyridine

On utilise la méthode de l'exemple 12 mais en partant de la dihydro-1,3 n-pentyle-3 (triméthoxy-3,4,5)-phényle-3 chlorométhyle-6 benzoxy-7 furo-(3,4-c)-pyridine. Le rendement est de 48% d'une poudre blanche fondant à 149°C (Tottoli), dont l'analyse élémentaire a montré une bonne correspondance avec la formule $C_{26}H_{36}N_2O_2S$.

Exemple 24

Dihydro-1,3 di-α-furyle-3,3 méthylaminométhyle-6 hydroxy-7 furo-(3,4-c)-pyridine

On utilise la méthode de l'exemple 12 mais en partant de la dihydro-1,3 di-α-furyle-3,3 chlorométhyle-6 benzoxy-7 furo-(3,4-c)-pyridine. Le rendement est de 62% d'une substance blanche cristallisée fondant à 176—179°C (Tottoli), dont l'analyse élémentaire a montré une bonne correspondance avec la formule $C_{17}H_{16}N_2O_4$, 2HCl.

Exemple 25

Dihydro-1,3 phényle-3 (triméthoxy-3,4,5)-phényléthyle-3 diméthylaminométhyle-6 hydroxy-7 furo-(3,4-c)-pyridine

On utilise la méthode de l'exemple 1 mais en partant de la dihydro-1,3 phényle-3 (triméthoxy-3,4,5)-phényléthyle-3 chlorométhyle-6 benzoxy-7 furo-(3,4-c)-pyridine. Le rendement est de 31% d'une substance grise fondant à 163—166°C (Tottoli), dont l'analyse élémentaire a montré une bonne correspondance avec la formule $C_{27}H_{32}N_2O_2$, 2HCl.

Exemple 26

Dihydro-1,3 méthyle-3 (p-pyrrolidinyléthoxy)-phényle-3 diméthylaminométhyle-6 hydroxy-7 furo-(3,4-c)-pyridine

On utilise la méthode de l'exemple 1 mais en partant de la dihydro-1,3 méthyle-3 (*p*-pyrrolidinyléthoxy)-phényle-3 chlorométhyle-6 benzoxy-7 furo-(3,4-c)-pyridine. Le rendement est de 54% d'une substance jaune pâle cristallisée fondant à 188°C (Tottoli), dont l'analyse élémentaire a montré une bonne correspondance avec la formule $C_{24}H_{33}N_3O_3$, 2HCl.

Exemple 27

Dihydro-1,3 p-toluyle-3 p-thiométhylphényle-3 pyrrolidinométhyle-6 hydroxy-7 furo-(3,4-c)-pyridine

On utilise la méthode de l'exemple 9 mais en partant de la dihydro-1,3 *p*-toluyle-3 *p*-thiométhylphényle-3 chlorométhyle-6 benzoxy-7 furo-(3,4-c)-pyridine. Le rendement est de 63% d'une substance jaune pâle cristallisée fondant à 174°C (Tottoli), dont l'analyse élémentaire a montré une très bonne correspondance avec la formule $C_{26}H_{28}N_2O_4S$.

TOXICITE

La toxicité a été recherchée sur le rat et la souris (toxicité aigüe per os). Comme les composés selon l'invention présentent une activité thérmapeutique à des doses inférieures à 25 mg/kg ou de cet ordre, on a administré une dose unique de 600 mg/kg par voie orale, mise en suspension dans un sirop de gomme, à des lots contenant 20 rats et 20 souris. Aucun décès n'a été constaté.

PHARMACOLOGIE

L'activité anti-allergique a été recherchée par le test de l'anaphylaxie cutanée passive sur des rats de souche Sprague-Dawley pesant 180 à 200 g chacun.

Le dos de chaque rat a été rasé et chaque animal a reçu (au temps — 48 heures) deux injections, chacune de 10 ml d'immunsérum homologue (dilution au quart); au temps — 30 minutes, chaque animal a reçu deux injections d'histamine (50 g/kg dissous dans 10 ml/kg de sérum), au temps 0, chaque animal a reçu une injection intraveineuse d'antigène (ovalbumine + Bleu Evans). Ceci a amené la formation de 4 papules cutanées caractérisées par leurs surfaces et la coloration qu'elles donnent après extraction après 48 heures par le formamide à 65°C. Le traitement qui a été décrit ci-dessus s'applique aux témoins. Pour les rats traités, on procède de la même façon, à cela près qu'au temps — 1 heure, on injecte une dose de 10 mg/kg du composé à tester.

Les lots étaient, chacun, de 6 animaux, tant pour les témoins, pour le composé de référence, la ketotifène, que pour chacun des composés testés (identifiés par leur numéro d'exemple de préparation).

Le traitement simultané par l'histamine était destiné à déterminer une éventuelle action anti-histaminique des composés selon l'invention. Une action anti-histaminique est un facteur peu favorable dans le traitement des allergies. Les résultats qui ont été reportés dans le tableau suivant montrent que les composés selon l'invention présentent une bonne activité anti-allergique, associée à une activité anti-histaminique favorablement basse, ce qui n'est pas le cas pour le composé de référence utilisé.

Dans le tableau sont donnés, pour chacun des composés testés, pour les papules à l'immunsérum et celles à l'histamine:

la surface des papules en mm2, suivie du pourcentage de réduction de cette surface par rapport aux témoins; ce pourcentage est suivi par *** pour un résultat hautement significatif, ** pour un résultat très significatif, * pour un résultat significatif, ou par N.S. pour un résultat non significatif.

les résultats de l'absorption colorimétrique par comparaison avec les témoins (en unités arbitraires), suivis du pourcentage de réduction par rapport aux témoins, avec la même convention en ce qui concerne le caractère significatif des résultats.

PRESENTATION — POSOLOGIE

Les composés selon l'invention peuvent être présentés en comprimés, gélules ou suspension pour administration par voie orale, chaque dosage unitaire contenant 250 mg d'ingrédient actif. En thérapeutique humaine, la posologie est de 2 à 6 doses par jour pour, environ, une semaine.

Par voie injectable, les ampoules contiennent 100 mg de principe actif et la posologie est de 1 à 5 ampoules par jour pour, environ, une semaine.

Tout excipient ou diluant usuel peut être employé pour les différentes préparations utilisées.

TABLEAU

| Composés | Papules à l'immunsérum | | Papules à l'histamine | |
|---|---|---|---|---|
| | Surface (mm2) % | Couleur % | Surface (mm2) % | Couleur % |
| Témoins | 84,4 ± 5,73 | 0,377 ± 0,0384 | 120,7 ± 7,40 | 1,213 ± 0,0726 |
| Ketotifène | 46,3 ± 6,26<br>- 45,1 *** | 0,226 ± 0,040<br>- 39,9 * | 53,4 ± 2,05<br>- 55,8 *** | 0,389 ± 0,0404<br>- 67,9 *** |
| 3 | 31,5 ± 7,98<br>- 63 *** | 0,166 ± 0,0354<br>- 56 *** | 95,9 ± 5,76<br>- 20 ** | 0,762 ± 0,0703<br>- 37 *** |
| 6 | 32 ± 8,01<br>- 62 *** | 0,184 ± 0,0372<br>- 51,2 *** | 112,7 ± 4,94<br>- 6,6 NS | 1,152 ± 0,0609<br>- 5 NS |
| 8 | 40,5 ± 9,02<br>- 52 *** | 0,236 ± 0,062<br>- 37,5 * | 103,2 ± 6,33<br>- 14,5 * | 1,025 ± 0,0525<br>- 15,4 NS |
| 9 | 41,6 ± 6,49<br>- 51 *** | 0,190 ± 0,031<br>- 50 *** | 103,8 ± 2,76<br>- 14 * | 0,892 ± 0,0426<br>- 26 ** |
| 10 | 34,5 ± 8,16<br>- 59 *** | 0,162 ± 0,0384<br>- 57 *** | 104,3 ± 4,71<br>- 14 NS | 0,931 ± 0,0755<br>- 23 ** |
| 11 | 42,1 ± 6,31<br>- 50 ** | 0,181 ± 0,0192<br>- 52 *** | 111,5 ± 6,8<br>- 7,6 NS | 1,029 ± 0,0499<br>- 15 NS |
| 12 | 32,3 ± 8,08<br>- 61,7 *** | 0,154 ± 0,0333<br>- 59,1 *** | 111 ± 6,64<br>- 8 NS | 0,922 ± 0,0544<br>- 24 NS |
| 15 | 49,1 ± 7,23<br>- 41,8 *** | 0,199 ± 0,0328<br>- 47 *** | 105,6 ± 7,06<br>- 12,5 NS | 1,076 ± 0,0873<br>- 11,3 NS |
| 20 | 23 ± 4,41<br>- 72,7 *** | 0,205 ± 0,0337<br>- 45,7 *** | 94,6 ± 5,05<br>- 21,6 ** | 1,035 ± 0,0912<br>- 14,7 NS |
| 21 | 35,6 ± 6,33<br>- 57,8 *** | 0,223 ± 0,0366<br>- 40,9 * | 107,9 ± 7,63<br>- 10,6 NS | 1,043 ± 0,0924<br>- 14 NS |
| 24 | 57,4 ± 4,12<br>- 32 ** | 0,194 ± 0,0195<br>- 48,5 *** | 127,6 ± 6,25<br>+ 5,7 NS | 1,128 ± 0,0946<br>- 7 NS |

**Revendications pour les Etats contractants: BE CH DE GB IT LI LU NL SE**

1. Dérivés de la dihydro-1,3 aminométhyle-6 hydroxy-7 furo-(3,4-c)-pyridine répondant à la formule générale I

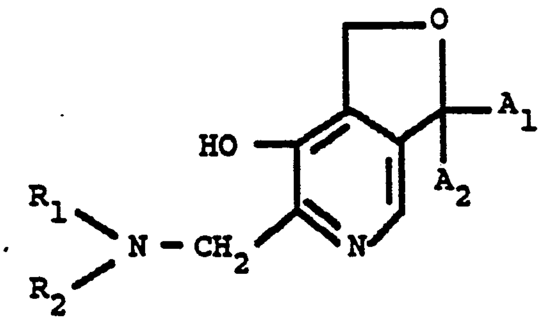

I

dans laquelle chacun des $A_1$ et $A_2$, indépendamment, représente un atome d'hydrogène, un groupement hydrocarboné saturé ou non saturé en chaîne droite ayant de 1 à 5 atomes de carbone, un groupement hétérocyclique ayant jusqu'à 6 atomes dans le cycle, chacun des groupements représentés par $A_1$ et $A_2$ étant non substitué ou substitué par un ou plusieurs atomes de chlore ou de fluor, groupements trifluorométhyle, groupements alcoyle ayant de 1 à 5 atomes de carbone, groupements alcoxy ayant de 1 à 5 atomes de carbone, groupements alcoylthio ayant de 1 à 5 atomes de carbone, groupements dialcoylamino dans lesquels chacun des groupements alcoyle a de 1 à 5 atomes de carbone, groupements dialcoylaminoalcoxy dans lesquels chacun des groupements alcoyle et des groupements alcoxy a de 1 à 5 atomes de carbone ou des groupements α- ou β-alcoxy-N-pyrrolidinyle dans lesquels le groupement alcoxy a de 1 à 5 atomes de carbone; $R_1$ ou $R_2$ représentent l'un un atome d'hydrogène, l'autre un groupement méthyle ou un groupement éthyle, ou $R_1$ et $R_2$, indépendamment, représentent un groupement méthyle ou un groupement éthyle ou, ensemble avec l'atome d'azote, forment un cycle pyrrolidine ou pipéridine ou morpholine ou thiomorpholine; ainsi que les dihydro-1,3 phényle-3 diméthylaminométhyle-6 hydroxy-7 furo-(3,4-c)-pyridine, dihydro-1,3 p-chlorophényle-3 diméthylaminométhyle-6 hydroxy-7 furo-(3,4-c)-pyridine, dihydro-1,3 p-fluorophényle-3 diméthylaminométhyle-6 hydroxy-7 furo-(3,4-c)-pyridine, dihydro-1,3 phényle-3 méthylaminométhyle-6 hydroxy-7 furo-(3,4-c)-pyridine, dihydro-1,3 p-chlorophényle-3 méthylaminométhyle-6 hydroxy-7 furo-(3,4-c)-pyridine, dihydro-1,3 p-fluorophényle-3 méthylaminométhyle-6 hydroxy-7 furo-(3,4-c)-pyridine, dihydro-1,3 phényle-3 pyrrolidinométhyle-6 hydroxy-7 furo-(3,4-c)-pyridine, dihydro-1,3 phényle-3 pipéridinométhyle-6 hydroxy-7 furo-(3,4-c)-pyridine, dihydro-1,3 phényle-3 morpholinométhyle-6 hydroxy-7 furo-(3,4-c)-pyridine, dihydro-1,3 phényle-3 thiomorpholinométhyle-6 hydroxy-7 furo-(3,4-c)-pyridine, dihydro-1,3 éthyle-3 p-chlorophényle-3 diméthylaminométhyle-6 hydroxy-7 furo-(3,4-c)-pyridine, dihydro-1,3 phényle-3 p-fluorophényle-3 diméthylaminométhyle-6 hydroxy-7 furo-(3,4-c)-pyridine, dihydro-1,3 α-furyle-3 phényle-3 méthylaminométhyle-6 hydroxy-7 furo-(3,4-c)-pyridine, dihydro-1,3 p-trifluorométhylphényle-3 p-chlorophényle-3 méthylaminométhyle-6 hydroxy-7 furo-(3,4-c)-pyridine, dihydro-1,3 α-thiényle-3 p-fluorophényle-3 méthylaminométhyle-6 hydroxy-7 furo-(3,4-c)-pyridine, dihydro-1,3 cyclohexyle-3 phényle-3 pyrrolidinométhyle-6 hydroxy-7 furo-(3,4-c)-pyridine, dihydro-1,3 méthyle-3 p-diméthylaminoéthoxyphényle-3 pipéridinométhyle-6 hydroxy-7 furo-(3,4-c)-pyridine, dihydro-1,3 n-pentyle-3 (triméthoxy-3,4,5)-phényle-3 thiomorpholinométhyle-6 hydroxy-7 furo-(3,4-c)-pyridine, dihydro-1,3 phényle-3 (triméthoxy-3,4,5)-phényléthyle-3 diméthylaminométhyle-6 hydroxy-7 furo-(3,4-c)-pyridine, dihydro-1,3 méthyle-3 (p-pyrrolidinyléthoxy)-phényle-3 diméthylaminométhyle-6 hydroxy-7 furo-(3,4-c)-pyridine, dihydro-1,3 p-toluyle-3 p-thiométhylphényle-3 pyrrolidinométhyle-6 hydroxy-7 furo-(3;4-c)-pyridine et leurs sels pharmaceutiquement acceptables.

2. Procédé de préparation des composés selon la revendication 1, consistant à faire réagir un dérivé chlorométhyle-6 benzoxy-7 répondant à la formule générale II

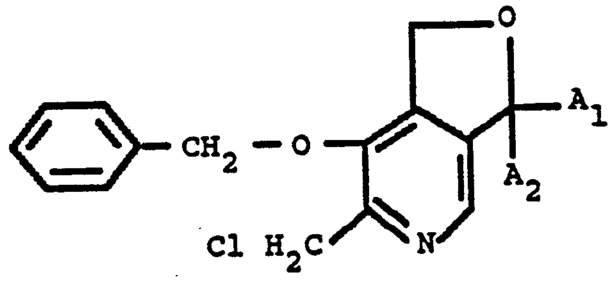

II

dans laquelle $A_1$ et $A_2$ ont la même signification que ci-dessus avec un excès d'un dérivé aminé répondant à la formule générale IV

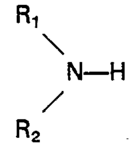

IV

dans laquelle $R_1$ et $R_2$ ont la même signification que ci-dessus, dans un solvant non polaire, à une température ne dépassant pas 20°C, ce qui conduit au dérivé portant la substitution désirée en position 6, puis à traiter ce composé par un acide pour débenzyliser le composé.

3. Une composition thérapeutique comprenant à titre d'ingrédient essentiel une quantité suffisante de l'un des composés selon la revendication 1, associé à tout diluant ou excipient approprié.

**Revendication pour l'Etat contractant: AT**

Procédé de préparation des nouveaux dérivés de la dihydro-1,3 aminométhyle-6 hydroxy-7 furo-(3,4-c)-pyridine répondant à la formule générale I

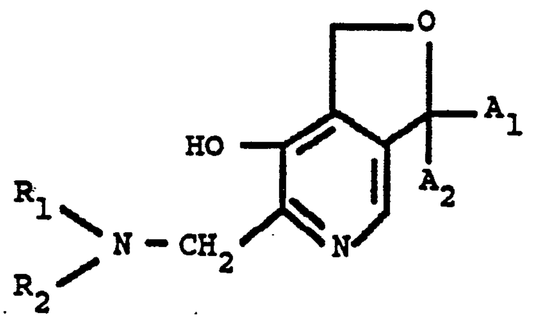

I

dans laquelle chacun des $A_1$ et $A_2$, indépendamment, représente un atome d'hydrogène, un groupement hydrocarboné saturé ou non saturé en chaîne droite ayant de 1 à 5 atomes de carbone, un groupement hétérocyclique ayant jusqu'à 6 atomes dans le cycle, chacun des groupements représentés par $A_1$ et $A_2$ étant non substitué ou substitué par un ou plusieurs atomes de chlore ou de fluor, groupements trifluorométhyle, groupements alcoyle ayant de 1 à 5 atomes de carbone, groupements alcoxy ayant de 1 à 5 atomes de carbone, groupements alcoylthio ayant de 1 à 5 atomes de carbone, groupements dialcoylamino dans lesquels chacun des groupements alcoyle a de 1 à 5 atomes de carbone, groupements dialcoylaminoalcoxy dans lesquels chacun des groupements alcoyle et des groupements alcoxy a de 1 à 5 atomes de carbone ou des groupements α- ou β-alcoxy-N-pyrrolidinyle dans lesquels le groupement alcoxy a de 1 à 5 atomes de carbone; $R_1$ ou $R_2$ représentent l'un un atome d'hydrogène, l'autre un groupement méthyle ou un groupement éthyle, ou $R_1$ et $R_2$, indépendamment, représentent un groupement méthyle ou un groupement éthyle, ou ensemble avec l'atome d'azote, forment un cycle pyrrolidine ou pipéridine ou morpholine ou thiomorpholine, ainsi que des dihydro-1,3 phényle-3 diméthylaminométhyle-6 hydroxy-7 furo-(3,4-c)-pyridine, dihydro-1,3 p-chlorophényle-3 diméthylaminométhyl-6 hydroxy-7 furo-(3,4-c)-pyridine, dihydro-1,3 p-fluorophényle-3 diméthylaminomethyle-6 hydroxy-7 furo-(3,4-c)-pyridine, dihydro-1,3 phényle-3 méthylaminométhyle-6 hydroxy-7 furo-(3,4-c)-pyridine, dihydro-1,3 p-chlorophényle-3 méthylaminométhyle-6 hydroxy-7 furo-(3,4-c)-pyridine, dihydro-1,3 p-fluorophényle-3 méthylaminométhyle-6 hydroxy-7 furo-(3,4-c)-pyridine, dihydro-1,3 phényle-3 pyrrolidinométhyle-6 hydroxy-7 furo-(3,4-c)-pyridine, dihydro-1,3 phényle-3 pipéridinométhyle-6 hydroxy-7 furo-(3,4-c)-pyridine, dihydro-1,3 phényle-3 morpholinométhyle-6 hydroxy-7 furo-(3,4-c)-pyridine, dihydro-1,3 phényle-3 thiomorpholinométhyle-6 hydroxy-7 furo-(3,4-c)-pyridine, dihydro-1,3 éthyle-3 p-chlorophényle-3 diméthylaminométhyle-6 hydroxy-7 furo-(3,4-c)-pyridine, dihydro-1,3 phényle-3 p-fluorophényle-3 diméthylaminométhyle-6 hydroxy-7 furo-(3,4-c)-pyridine, dihydro-1,3 α-furyle-3 phényle-3 méthylaminométhyle-6 hydroxy-7 furo-(3,4-c)-pyridine, dihydro-1,3 p-trifluorométhylphényle-3 p-chlorophényle-3 méthylaminométhyle-6 hydroxy-7 furo-(3,4-c)-pyridine, dihydro-1,3 α-thiényle-3 p-fluorophényle-3 méthylaminométhyle-6 hydroxy-7 furo-(3,4-c)-pyridine, dihydro-1,3 cyclohexyle-3 phényle-3 pyrrolidinométhyle-6 hydroxy-7 furo-(3,4-c)-pyridine, dihydro-1,3 méthyle-3 p-diméthylaminoéthoxyphényle-3 pipéridinométhyle-6 hydroxy-7 furo-(3,4-c)-pyridine, dihydro-1,3 n-pentyle-3 (triméthoxy-3,4,5)-phényle-3 thiomorpholinométhyle-6 hydroxy-7 furo-(3,4-c)-pyridine, dihydro-1,3 phényle-3 (triméthoxy-3,4,5)-phényléthyle-3 diméthylaminométhyle-6 hydroxy-7 furo-(3,4-c)-pyridine, dihydro-1,3 méthyle-3 (p-pyrrolidinyléthoxy)-phényle-3 diméthylaminométhyle-6 hydroxy-7 furo-(3,4-c)-pyridine, dihydro-1,3 p-toluyle-3 p-thiométhylphényle-3 pyrrolidinométhyle-6 hydroxy-7 furo-(3,4-c)-pyridine et de leurs sels pharmaceutiquement acceptables, consistant à faire réagir un dérivé chlorométhyle-6 benzoxy-7 répondant à la formule générale II

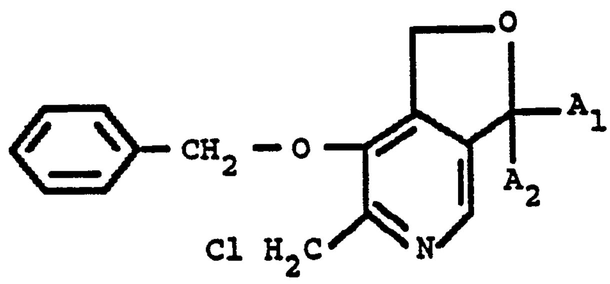

II

dans laquelle $A_1$ et $A_2$ ont la même signification que ci-dessus avec un excès d'un dérivé aminé répondant à la formule générale IV

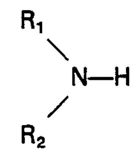

IV

dans laquelle $R_1$ et $R_2$ ont la même signification que ci-dessus, dans un solvant non polaire, à une température ne dépassant pas 20°C, ce qui conduit au dérivé portant la substitution désirée en position 6, puis à traiter ce composé par un acide pour débenzyliser le composé.

**Patentansprüche für die Vertragsstaaten: BE CH DE GB IT LI LU NL SE**

1. 1,3-Dihydro-6-aminomethyl-7-hydroxyfuro(3,4-c)pyridinderivate der allgemeinen Formel I

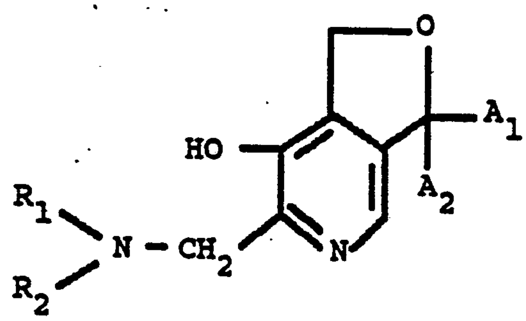

I

worin jedes von $A_1$ und $A_2$ unabhängig ein Wasserstoffatom, eine gesättigte oder nicht gesättigte geradkettige Kohlenwasserstoffgruppe mit 1 bis 5 Kohlenstoffatomen, eine heterocyclische Gruppe mit bis zu 6 Atomen im Ring, wobei jede durch $A_1$ und $A_2$ wiedergegebene Gruppe unsubstituiert oder durch ein oder mehrere Chlor- oder Fluoratome, Trifluormethylgruppen, Alkoylgruppen mit 1 bis 5 Kohlenstoffatomen, Alkoxygruppen mit 1 bis 5 Kohlenstoffatomen, Alkoylthiogruppen mit 1 bis 5 Kohlenstoffatomen, Dialkoylaminogruppen, in welchen jede Alkoylgruppe 1 bis 5 Kohlenstoffatome hat, Dialkoylaminoalkoxygruppen, in denen jede Alkoylgruppe und Alkoxygruppe 1 bis 5 Kohlenstoffatome hat, oder α- oder β-Alkoxy-N-Pyrrolidinylgruppen, in denen die Alkoxygruppe 1 bis 5 Kohlenstoffatome hat, substituiert ist, darstellt; das eine von $R_1$ oder $R_2$ ein Wasserstoffatom, das andere eine Methylgruppe oder eine Ethylgruppe darstellt oder $R_1$ und $R_2$ unabhängig eine Methylgruppe oder eine Ethylgruppe darstellen oder zusammen mit dem Stickstoffatom einen Pyrrolidin- oder Piperidin- oder Morpholin- oder Thiomorpholinring bilden; sowie 1,3 - Dihydro - 3 - phenyl - 6 - dimethylaminomethyl - 7 - hydroxyfuro(3,4-c)pyridin, 1,3 - Dihydro - 3 - p - chlorphenyl - 6 - dimethylaminomethyl - 7 - hydroxyfuro(3,4-c)pyridin, 1,3 - Dihydro - 3 - p - fluorphenyl - 6 - dimethylaminomethyl - 7 - hydroxyfuro(3,4-c)pyridin, 1,3 - Dihydro - 3 - phenyl - 6 - methylaminomethyl - 7 - hydroxyfuro(3,4-c)pyridin, 1,3 - Dihydro - 3 - p - chlorphenyl - 6 - methylaminomethyl - 7 - hydroxyfuro(3,4-c)pyridin, 1,3 - Dihydro - 3 - p - fluorphenyl - 6 - methylaminomethyl - 7 - hydroxyfuro(3,4-c)pyridin, 1,3 - Dihydro - 3 - phenyl - 6 - pyrrolidinomethyl - 7 - hydroxyfuro(3,4-c)pyridin, 1,3 - Dihydro - 3 - phenyl - 6 - piperidinomethyl - 7 - hydroxyfuro(3,4-c)-pyridin, 1,3 - Dihydro - 3 - phenyl - 6 - morpholinomethyl - 7 - hydroxyfuro(3,4-c)pyridin, 1,3 - Dihydro - 3 - phenyl - 6 - thiomorpholinomethyl - 7 - hydroxyfuro(3,4-c)pyridin, 1,3 - Dihydro - 3 - ethyl - 3 - p - chlorphenyl - 6 - dimethylaminomethyl - 7 - hydroxyfuro(3,4-c)pyridin, 1,3 - Dihydro - 3 - phenyl - 3 - p - fluorphenyl - 6 - dimethylaminomethyl - 7 - hydroxyfuro(3,4-c)pyridin, 1,3 - Dihydro - 3 - α - furyl - 3 - phenyl - 6 - methylaminomethyl - 7 - hydroxyfuro(3,4-c)pyridin, 1,3 - Dihydro - 3 - p - trifluormethylphenyl - 3 - p - chlorphenyl - 6 - methylaminomethyl - 7 - hydroxyfuro(3,4-c)pyridin, 1,3 -

Dihydro - 3 - α - thienyl - 3 - p - fluorphenyl - 6 - methylaminomethyl - 7 - hydroxyfuro(3,4-c)pyridin, 1,3 - Dihydro - 3 - cyclohexyl - 3 - phenyl - 6 - pyrrolidinomethyl - 7 - hydroxyfuro(3,4-c)pyridin, 1,3 - Dihydro - 3 - methyl - 3 - p - dimethylaminoethoxyphenyl - 6 - piperidinomethyl - 7 - hydroxyfuro(3,4-c)pyridin, 1,3 - Dihydro - 3 - n - pentyl - 3 - (3,4,5 - trimethoxy) - phenyl - 6 - thiomorpholinomethyl - 7 - hydroxyfuro(3,4-c)pyridin, 1,3 - Dihydro - 3 - phenyl - 3 - (3,4,5 - trimethoxy)phenylethyl - 6 - dimethylaminomethyl - 7 - hydroxyfuro(3,4-c)pyridin, 1,3 - Dihydro - 3 - methyl - 3 - (p - pyrrolidinylethoxy)phenyl - 6 - dimethylaminomethyl - 7 - hydroxyfuro(3,4-c)pyridin, 1,3 - Dihydro - 3 - p - toluyl - 3 - p - thiomethylphenyl - 6 - pyrrolidinomethyl - 7 - hydroxyfuro(3,4-c)pyridin und ihre pharmazeutisch annehmbaren Salze.

2. Verfahren zur Herstellung der Verbindungen nach Anspruch 1, bestehend in der Umsetzung eines 6-Chlormethyl-7-benzoxyderivats der allgemeinen Formel II

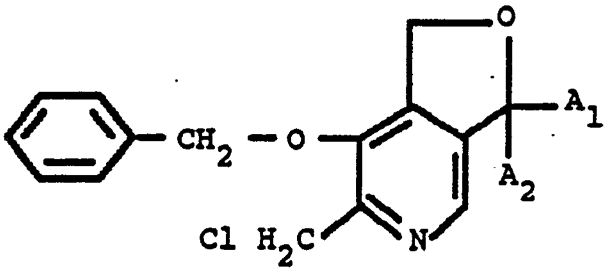

II

worin $A_1$ und $A_2$ die gleiche Bedeutung haben wie oben, mit einem Überschuß eines Aminderivats der allgemeinen Formel IV

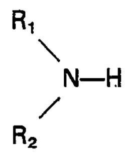

IV

worin $R_1$ und $R_2$ die gleiche Bedeutung haben wie oben, in einem nicht polaren Lösungsmittel bei einer Temperatur, die 20°C nicht überschreitet, was zu einem Derivat mit der erwünschten Substitution in der 6-Stellung führt, und danach Behandeln der Verbindung mit einer Säure zur Debenzylisierung der Verbindung.

3. Therapeutische Zusammensetzung, welche als wesentlichen Bestandteil eine ausreichende Menge einer der Verbindungen nach Anspruch 1 umfaßt, assoziiert mit jedem geeigneten Verdünnungsmittel oder Exipienten.

**Patentanspruch für den Vertragsstaat: AT**

Verfahren zur Herstellung von neuen 1,3-Dihydro-6-aminomethyl-7-hydroxyfuro(3,4-c)pyridinderivaten der allgemeinen Formel I

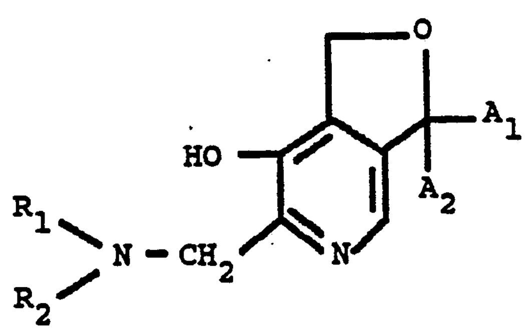

I

worin jedes von $A_1$ und $A_2$ unabhängig ein Wasserstoffatom, eine gesättigte oder nicht gesättigte geradkettige Kohlenwasserstoffgruppe mit 1 bis 5 Kohlenstoffatomen, eine heterocyclische Gruppe mit bis zu 6 Atomen im Ring, wobei jede durch $A_1$ und $A_2$ wiedergegebene Gruppe unsubstituiert oder durch ein oder mehrere Chlor- oder Fluoratome, Trifluormethylgruppen, Alkoylgruppen mit 1 bis 5 Kohlenstoffatomen, Alkoxygruppen mit 1 bis 5 Kohlenstoffatomen, Alkoylthiogruppen mit 1 bis 5 Kohlenstoffatomen, Dialkoylaminogruppen, in welchen jede Alkoylgruppe 1 bis 5 Kohlenstoffatome hat, Dialkoylaminoalkoxygruppen, in denen jede Alkoylgruppe und Alkoxygruppe 1 bis 5 Kohlenstoffatome hat, oder α- oder β-

Alkoxy-N-Pyrrolidinylgruppen, in denen die Alkoxygruppe 1 bis 5 Kohlenstoffatome hat, substituiert ist, darstellt; das eine von $R_1$ oder $R_2$ ein Wasserstoffatom, das andere eine Methylgruppe oder eine Ethylgruppe darstellt oder $R_1$ und $R_2$ unabhängig eine Methylgruppe oder eine Ethylgruppe darstellen oder zusammen mit dem Stickstoffatom einen Pyrrolidin- oder Piperidin- oder Morpholin- oder Thiomorpholinring bilden; sowie 1,3 - Dihydro - 3 - phenyl - 6 - dimethylaminomethyl - 7 - hydroxyfuro(3,4-c)pyridin, 1,3 - Dihydro - 3 - p - chlorphenyl - 6 - dimethylaminomethyl - 7 - hydroxyfuro(3,4-c)pyridin, 1,3 - Dihydro - 3 - p - fluorphenyl - 6 - dimethylaminomethyl - 7 - hydroxyfuro(3,4-c)pyridin, 1,3 - Dihydro - 3 - phenyl - 6 - methylaminomethyl - 7 - hydroxyfuro(3,4-c)pyridin, 1,3 - Dihydro - 3 - p - chlorphenyl - 6 - methylaminomethyl - 7 - hydroxyfuro(3,4-c)pyridin, 1,3 - Dihydro - 3 - p - fluorphenyl - 6 - methylaminomethyl - 7 - hydroxyfuro(3,4-c)pyridin, 1,3 - Dihydro - 3 - phenyl - 6 - pyrrolidinomethyl - 7 - hydroxyfuro(3,4-c)pyridin, 1,3 - Dihydro - 3 - phenyl - 6 - piperidinomethyl - 7 - hydroxyfuro(3,4-c)-pyridin, 1,3 - Dihydro - 3 - phenyl - 6 - morpholinomethyl - 7 - hydroxyfuro(3,4-c)pyridin, 1,3 - Dihydro - 3 - phenyl - 6 - thiomorpholinomethyl - 7 - hydroxyfuro(3,4-c)pyridin, 1,3 - Dihydro - 3 - ethyl - 3 - p - chlorphenyl - 6 - dimethylaminomethyl - 7 - hydroxyfuro(3,4-c)pyridin, 1,3 - Dihydro - 3 - phenyl - 3 - p - fluorphenyl - 6 - dimethylaminomethyl - 7 - hydroxyfuro(3,4-c)pyridin, 1,3 - Dihydro - 3 - α - furyl - 3 - phenyl - 6 - methylaminomethyl - 7 - hydroxyfuro(3,4-c)pyridin, 1,3 - Dihydro - 3 - p - trifluormethylphenyl - 3 - p - chlorphenyl - 6 - methylaminomethyl - 7 - hydroxyfuro(3,4-c)pyridin, 1,3 - Dihydro - 3 - α - thienyl - 3 - p - fluorphenyl - 6 - methylaminomethyl - 7 - hydroxyfuro(3,4-c)pyridin, 1,3 - Dihydro - 3 - cyclohexyl - 3 - phenyl - 6 - pyrrolidinomethyl - 7 - hydroxyfuro(3,4-c)pyridin, 1,3 - Dihydro - 3 - methyl - 3 - p - dimethylaminoethoxyphenyl - 6 - piperidinomethyl - 7 - hydroxyfuro(3,4-c)pyridin, 1,3 - Dihydro - 3 - n - pentyl - 3 - (3,4,5 - trimethoxy) - phenyl - 6 - thiomorpholinomethyl - 7 - hydroxyfuro(3,4-c)pyridin, 1,3 - Dihydro - 3 - phenyl - 3 - (3,4,5 - trimethoxy)phenylethyl - 6 - dimethylaminomethyl - 7 - hydroxyfuro(3,4-c)pyridin, 1,3 - Dihydro - 3 - methyl - 3 - (p - pyrrolidinylethoxy)phenyl - 6 - dimethylaminomethyl - 7 - hydroxyfuro(3,4-c)pyridin, 1,3 - Dihydro - 3 - p - toluyl - 3 - p - thiomethylphenyl - 6 - pyrrolidinomethyl - 7 - hydroxyfuro(3,4-c)pyridin und ihre pharmazeutisch annehmbaren Salze, bestehend in der Umsetzung eines 6-Chlormethyl-7-benzoxyderivats der allgemeinen Formel II

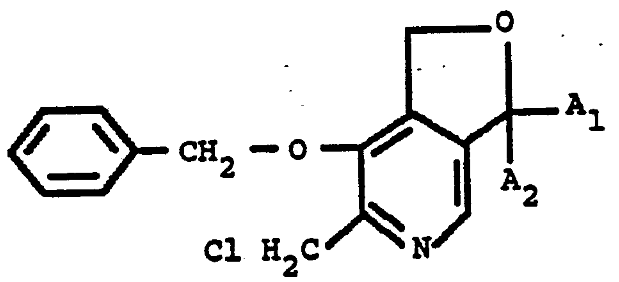

II

worin $A_1$ und $A_2$ die gleiche Bedeutung haben wie oben, mit einem Überschuß eines Aminderivats der allgemeinen Formel IV

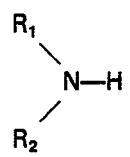

IV

worin $R_1$ und $R_2$ die gleiche Bedeutung haben wie oben, in einem nicht polaren Lösungsmittel bei einer Temperatur, die 20°C nicht überschreitet, was zu einem Derivat mit der erwünschten Substitution der 6-Stellung führt, und danach Behandeln der Verbindung mit einer Säure zur Debenzylisierung der Verbindung.

**Claims for the Contracting States: BE CH DE GB IT LI LU NL SE**

1. Derivatives of 1,3-dihydro-6-aminomethyl-7-hydroxy-furo-(3,4-c)-pyridine satisfying the general formula I

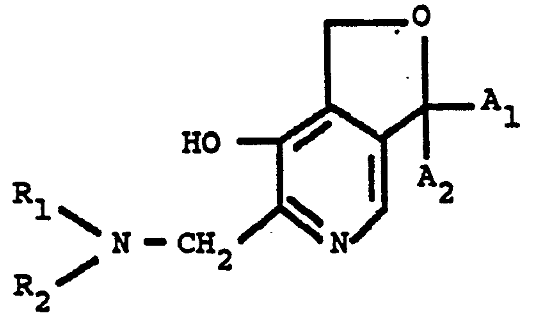

I

in which each of $A_1$ and $A_2$, independently, represents a hydrogen atom, a saturated or unsaturated straight chain hydrocarbon group having from 1 to 5 carbon atoms, a heterocyclic group having up to 6 ring atoms, each of the groups represented by $A_1$ and $A_2$ being unsubstituted or substituted by one or more chlorine or fluorine atoms, trifluoromethyl groups, alkyl groups having from 1 to 5 carbon atoms, alkoxy groups having from 1 to 5 carbon atoms, alkylthio groups having from 1 to 5 carbon atoms, dialkylamino groups in which each alkyl group has from 1 to 5 carbon atoms, dialkylaminoalkoxy groups in which each alkyl group and alkoxy group has from 1 to 5 carbon atoms or α- or β-alkoxy-N-pyrrolidinyl groups in which the alkoxy group has from 1 to 5 carbon atoms; one of $R_1$ or $R_2$ represents a hydrogen atom, the other a methyl or ethyl group, or $R_1$ and $R_2$, independently, represents a methyl group or an ethyl group or, together with the nitrogen atom, form a pyrrolidine or piperidine or morpholine or thiomorpholine ring; as well as 1,3 - dihydro - 3 - phenyl - 6 - dimethylaminomethyl - 7 - hydroxy - furo - (3,4-c) - pyridine, 1,3 - dihydro - 3 - (p - chlorophenyl) - 6 - dimethylaminomethyl - 7 - hydroxy - furo - (3,4-c) - pyridine, 1,3 - dihydro - 3 - (p - fluorophenyl) - 6 - dimethylaminomethyl - 7 - hydroxy - furo - (3,4-c) - pyridine, 1,3 - dihydro - 3 - phenyl - 6 - methylaminomethyl - 7 - hydroxy - furo - (3,4-c) - pyridine, 1,3 - dihydro - 3 - (p - chlorophenyl) - 6 - methylaminomethyl - 7 - hydroxy - furo - (3,4-c) - pyridine, 1,3 - dihydro - 3 - (p - fluorophenyl) - 6 - methylaminomethyl - 7 - hydroxy - furo - (3,4-c) - pyridine, 1,3 - dihydro - 3 - phenyl - 6 - pyrrolidinomethyl - 7 - hydroxy - furo - (3,4-c) - pyridine, 1,3 - dihydro - 3 - phenyl - 6 - piperidinomethyl - 7 - hydroxy - furo - (3,4-c) - pyridine, 1,3 - dihydro - 3 - phenyl - 6 - morpholinomethyl - 7 - hydroxy - furo - (3,4-c) - pyridine, 1,3 - dihydro - 3 - phenyl - 6 - thiomorpholinomethyl - 7 - hydroxy - furo - (3,4-c) - pyridine, 1,3 - dihydro - 3 - ethyl - 3 - (p - chlorophenyl) - 6 - dimethylaminomethyl - 7 - hydroxy - furo - (3,4-c) - pyridine, 1,3 - dihydro - 3 - phenyl - 3 - (p - fluorophenyl) - 6 - dimethylaminomethyl - 7 - hydroxy - furo - (3,4-c) - pyridine, 1,3 - dihydro - 3 - (α - furyl) - 3 - phenyl - 6 - methylaminomethyl - 7 - hydroxy - furo - (3,4-c) - pyridine, 1,3 - dihydro - 3 - (p - trifluoromethylphenyl) - 3 - (p - chlorophenyl) - 6 - methylaminomethyl - 7 - hydroxy - furo - (3,4-c) - pyridine, 1,3 - dihydro - 3 - (α - thienyl) - 3 - (p - fluorophenyl) - 6 - methylaminomethyl - 7 - hydroxy - furo - (3,4-c) - pyridine, 1,3 - dihydro - 3 - cyclohexyl - 3 - phenyl - 6 - pyrrolidinomethyl - 7 - hydroxy - furo - (3,4-c) - pyridine, 1,3 - dihydro - 3 - methyl - 3 - (p - dimethylaminoethoxyphenyl) - 6 - piperidinomethyl - 7 - hydroxy - furo - (3,4-c) - pyridine, 1,3 - dihydro - 3 - (n - pentyl) - 3 - (3,4,5 - trimethoxyphenyl) - 6 - thiomorpholinomethyl - 7 - hydroxy - furo - (3,4-c) - pyridine, 1,3 - dihydro - 3 - phenyl - 3 - (3,4,5 - trimethoxy - phenylethyl) - 6 - dimethylaminomethyl - 7 - hydroxy - furo - (3,4-c) - pyridine, 1,3 - dihydro - 3 - methyl - 3 - (p - pyrrolidinylethoxy - phenyl) - 6 - dimethylaminomethyl - 7 - hydroxy - furo - (3,4-c) - pyridine, 1,3 - dihydro - 3 - (p - toluyl) - 3 - (p - thiomethylphenyl) - 6 - pyrrolidinomethyl - 7 - hydroxy - furo - (3,4-c) - pyridine and their pharmaceutically acceptable salts.

2. A process of preparation of the compounds according to claim 1, consisting of reacting a 6-chloromethyl-7-benzoxy derivative of the general formula II

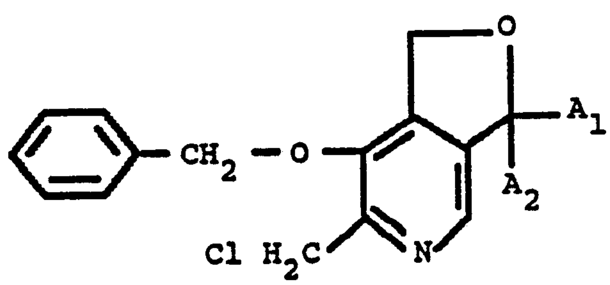

II

in which $A_1$ and $A_2$ have the same meaning as hereinabove with an excess of an amino derivative satisfying the general formula IV

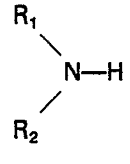

IV

in which $R_1$ and $R_2$ have the same meaning as hereinabove, in a non-polar solvent, at a temperature not exceeding 20°C, which leads to a derivative bearing the desired substitution in position 6, then treating this compound with an acid to debenzylise the compound.

3. A therapeutic composition comprising as an essential ingredient a sufficient amount of one of the compounds according to claim 1, in association with an appropriate diluant or excipient.

**Claim for the Contracting State: AT**

A process of preparation of new derivatives of 1,3-dihydro-6-aminomethyl-7-hydroxy-furo-(3,4-c)-pyridine satisfying the general formula I

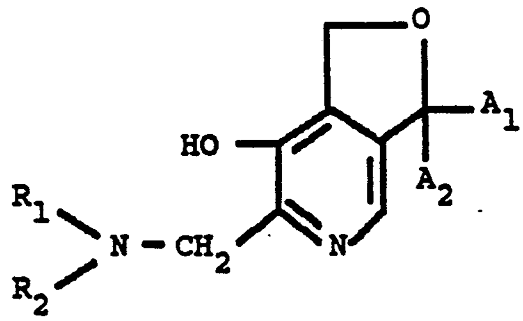

I

in which each of $A_1$ and $A_2$, independently, represents a hydrogen atom, a saturated or unsaturated straight chain hydrocarbon group having from 1 to 5 carbon atoms, a heterocyclic group having up to 6 ring atoms, each of the groups represented by $A_1$ and $A_2$ being unsubstituted or substituted by one or more chlorine or fluorine atoms, trifluoromethyl groups, alkyl groups having from 1 to 5 carbon atoms, alkoxy groups having from 1 to 5 carbon atoms, alkylthio groups having from 1 to 5 carbon atoms, dialkylamino groups in which each alkyl group has from 1 to 5 carbon atoms, dialkylaminoalkoxy groups in which each alkyl group and alkoxy group has from 1 to 5 carbon atoms or α- or β-alkoxy-N-pyrrolidinyl groups in which the alkoxy group has from 1 to 5 carbon atoms; one of $R_1$ or $R_2$ represents a hydrogen atom, the other a methyl or ethyl group, or $R_1$ and $R_2$, independently, represents a methyl group or an ethyl group or, together with the nitrogen atom, form a pyrrolidine or piperidine or morpholine or thiomorpholine ring; as well as of 1,3 - dihydro - 3 - phenyl - 6 - dimethylaminomethyl - 7 - hydroxy - furo - (3,4-c) - pyridine, 1,3 - dihydro - 3 - (p - chlorophenyl) - 6 - dimethylaminomethyl - 7 - hydroxy - furo - (3,4-c) - pyridine, 1,3 - dihydro - 3 - (p - fluorophenyl) - 6 - dimethylaminomethyl - 7 - hydroxy - furo - (3,4-c) - pyridine, 1,3 - dihydro - 3 - phenyl - 6 - methylaminomethyl - 7 - hydroxy - furo - (3,4-c) - pyridine, 1,3 - dihydro - 3 - (p - chlorophenyl) - 6 - methylaminomethyl - 7 - hydroxy - furo - (3,4-c) - pyridine, 1,3 - dihydro - 3 - (p - fluorophenyl) - 6 - methylaminomethyl - 7 - hydroxy - furo - (3,4-c) - pyridine, 1,3 - dihydro - 3 - phenyl - 6 - pyrrolidinomethyl - 7 - hydroxy - furo - (3,4-c) - pyridine, 1,3 - dihydro - 3 - phenyl - 6 - piperidinomethyl - 7 - hydroxy - furo - (3,4-c) - pyridine, 1,3 - dihydro - 3 - phenyl - 6 - morpholinomethyl - 7 - hydroxy - furo - (3,4-c) - pyridine, 1,3 - dihydro - 3 - phenyl - 6 - thiomorpholinomethyl - 7 - hydroxy - furo - (3,4-c) - pyridine, 1,3 - dihydro - 3 - ethyl - 3 - (p - chlorophenyl) - 6 - dimethylaminomethyl - 7 - hydroxy - furo - (3,4-c) - pyridine, 1,3 - dihydro - 3 - phenyl - 3 - (p - fluorophenyl) - 6 - dimethylaminomethyl - 7 - hydroxy - furo - (3,4-c) - pyridine, 1,3 - dihydro - 3 - (α - furyl) - 3 - phenyl - 6 - methylaminomethyl - 7 - hydroxy - furo - (3,4-c) - pyridine, 1,3 - dihydro - 3 - (p - trifluoromethylphenyl) - 3 - (p - chlorophenyl) - 6 - methylaminomethyl - 7 - hydroxy - furo - (3,4-c) - pyridine, 1,3 - dihydro - 3 - (α - thienyl) - 3 - (p - fluorophenyl) - 6 - methylaminomethyl - 7 - hydroxy - furo - (3,4-c) - pyridine, 1,3 - dihydro - 3 - cyclohexyl - 3 - phenyl - 6 - pyrrolidinomethyl - 7 - hydroxy - furo - (3,4-c) - pyridine, 1,3 - dihydro - 3 - methyl - 3 - (p - dimethylaminoethoxyphenyl) - 6 - piperidinomethyl - 7 - hydroxy - furo - (3,4-c) - pyridine, 1,3 - dihydro - 3 - (n - pentyl) - 3 - (3,4,5 - trimethoxyphenyl) - 6 - thiomorpholinomethyl - 7 - hydroxy - furo - (3,4-c) - pyridine, 1,3 - dihydro - 3 - phenyl - 3 - (3,4,5 - trimethoxy - phenylethyl) - 6 - dimethylaminomethyl - 7 - hydroxy - furo - (3,4-c) - pyridine, 1,3 - dihydro - 3 - methyl - 3 - (p - pyrrolidinylethoxy - phenyl) - 6 - dimethylaminomethyl - 7 - hydroxy - furo - (3,4-c) - pyridine, 1,3 - dihydro - 3 - (p - toluyl) - 3 - (p - thiomethylphenyl) - 6 - pyrrolidinomethyl - 7 - hydroxy - furo - (3,4-c) - pyridine and their pharmaceutically acceptable salts, consisting of reacting a 6-chloromethyl-7-benzoxy derivative of the general formula II

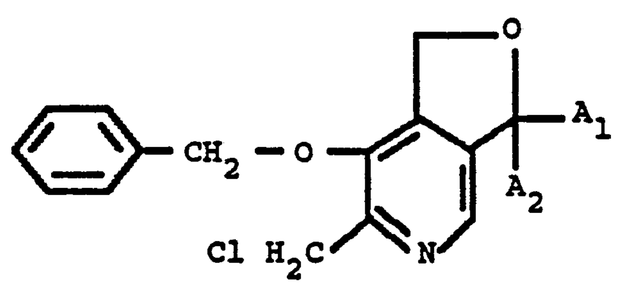

II

in which $A_1$ and $A_2$ have the same meaning as hereinabove with an excess of an amino derivative satisfying the general formula IV

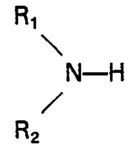

IV

in which $R_1$ and $R_2$ have the same meaning as hereinabove, in a non-polar solvent, at a temperature not exceeding 20°C, which leads to a derivative bearing the desired substitution in position 6, then treating this compound with an acid to debenzylise the compound.